# EUROPÄISCHE PATENTSCHRIFT

(11) **EP 2 125 053 B1**
(45) Veröffentlichungstag und Bekanntmachung des Hinweises auf die Patenterteilung: **04.03.2015**
(21) Anmeldenummer: 08701072.4
(22) Anmeldetag: 11.01.2008
(51) Int. Cl.: A61L 27/36, A61L 2/18, A61L 27/50

(54) **VERFAHREN ZUR BEHANDLUNG BIOLOGISCHER GEWEBE TIERISCHEN ODER MENSCHLICHEN URSPRUNGS WIE SCHWEINE-, RINDERPERIKARD- ODER MENSCHLICHER LEICHEN-HERZKLAPPEN SOWIE ENTSPRECHEND BEHANDELTES BIOLOGISCHES GEWEBE**
METHOD FOR THE TREATMENT OF BIOLOGICAL TISSUE OF ANIMAL OR HUMAN ORIGIN, SUCH AS PORCINE OR BOVINE PERICARDIUM OR HUMAN CADAVER HEART VALVES, AND BIOLOGICAL TISSUE TREATED ACCORDINGLY
PROCÉDÉ DE TRAITEMENT DE TISSUS BIOLOGIQUES D'ORIGINE ANIMALE OU HUMAINE TELS QUE DES VALVES PÉRICARDIQUES DE PORC OU DE VEAU OU DES VALVULES CARDIAQUES PRÉLEVÉES SUR DES DONNEURS HUMAINS DÉCÉDÉS, ET TISSU BIOLOGIQUE TRAITÉ EN CONSÉQUENCE

(30) Priorität: 24.02.2007 DE 102007009095
(43) Veröffentlichungstag der Anmeldung: 02.12.2009
(73) Patentinhaber: pfm medical titanium gmbh, 90431 Nürnberg (DE)
(72) Erfinder: ZIMMERMANN, Hanngörg, 91327 Gössweinstein (DE); HEINLEIN, Markus, 91327 Gössweinstein (DE)
(74) Vertreter: Hübner, Gerd
(86) Internationale Anmeldenummer: PCT/EP2008/000155
(87) Internationale Veröffentlichungsnummer: WO 2008/101566

(56) Entgegenhaltungen:
- WO-A-01/91820
- WO-A-03/075790
- WO-A-2006/077582
- US-A- 6 093 530

## Beschreibung

Die Erfindung betrifft ein Verfahren zur Behandlung biologischer Gewebe tierischen und menschlichen Ursprungs, wie Schweineherzklappen, Herzklappen aus Rinderperikard oder menschlicher Leichen-Herzklappen, sowie ein entsprechend behandeltes biologisches Gewebe.

Zum Hintergrund der Erfindung ist festzuhalten, dass Operationsverfahren mit einer Anwendung unterschiedlich vorbehandelter biologischer Gewebe tierischen Ursprungs als kollagene Matrizen insbesondere im Rahmen des Tissue-Engineerings in verschiedenen chirurgischen Teilgebieten an Bedeutung für künftige, verbesserte Therapieverfahren gewinnen. Einschlägige Anwendungsgebiete sind in der kardiovaskulären Chirurgie zu sehen, aber auch die Orthopädie und Neurochirurgie sind als Einsatzbereiche denkbar.

Für die Anwendung kollagener Matrices in der kardiovaskulären Chirurgie müssen eine gute Blutverträglichkeit und mechanische Stabilität gewährleistet sein. Als Beispiel sind in diesem Zusammenhang Herzklappen tierischen Ursprungs, wie Schweineklappen oder Klappen aus Rinderperikard, genauso zu nennen, wie biologische Gefäßprothesen mit kleinen Durchmessern und Pumpkammern biologischer oder mechanischer Blutpumpen. Bei orthopädisch-chirurgischen Behandlungen sind besonders stabile Kollagenmatrices zum Ersatz von Knorpeln, Bändern und Sehnen von besonderem Interesse. In der Neurochirurgie schließlich ist ein kollagenes Gewebe zum Verschluss des Hirnschädels etwa nach Tumoroperationen als Einsatzgebiet der vorliegenden Erfindung ins Auge zu fassen.

Die spezielle Problematik, wie sie der Erfindung zugrunde liegt, ist am Beispiel von Ersatzherzklappen deutlich zu machen. So entfallen von den weltweit jährlich bei über 200 000 Patienten implantierten Ersatzherzklappen etwa 50 % auf künstliche, mechanische Herzklappen und 50 % auf biologische Implantate auf der Basis von Schweineherzklappen und Klappen aus Rinderperikard. Die Implantation mechanischer Herzklappen erfordert zur dauerhaften Nachsorge die Verabreichung von blutgerinnungshemmenden Medikamenten, um von den Prothesen ausgehende Embolien zu vermeiden. Damit werden derart versorgte Patienten quasi zu "künstlichen Blutern".

Biologische Herzklappen tierischen Ursprungs haben das Problem, dass sie zur Erzielung einer Langzeitstabilität mit Glutaraldehyd behandelt werden müssen. Durch die dabei entstehenden freien Aldehyd-Gruppen aus Glutardialdehyd wirken die biologischen Herzklappen an sich toxisch und können somit nicht mit Zellen besiedelt werden. Eine Zellbesiedlung sollte diese Bioklappen bedeutend länger haltbar machen. Vor einer Zellbesiedlung wäre jedoch eine Detoxifizierung notwendig. Aus dem Stand der Technik sind in diesem Zusammenhang medizinische Studien bekannt, bei denen Substanzen zum Einsatz kommen, die freie Aldehyd-Gruppen binden. Es wird in diesem Zusammenhang auf die folgenden Literaturstellen verwiesen:
Gott J.P., Chih P., Dorsey L., Jay J.L., Jett G.K., Schoen F.J., Girardot J.M., Guyton R.A. "Calcification of porcine valves: a successful new method of antimineralization" in Ann Thorac Surg 1992;53:207-216; Jones M., Eidbo E.E., Hilbert S.L., Ferrans V.J., Clarck R.E. "Anticalcification treatments of bioprosthetic heart valves: in vivo studies in sheep" in J Cardiovasc Surg 1989;4:69-73; Grabenwöger M., Sider J., Fitzal F., Zelenka C., Windberger U., Grimm M. "Impact of glutaraldehyde on calcification of pericardial bioprosthetic heart valve material" in Ann Thorac Surg 1996;62:772-7 und schließlich Webb C.L., Benedict J.J., Schoen F.J., Linden J.A., Levy R.J. "Inhibition of bioprosthetic valve calcification with aminodiphosphonate covalently bound material to residual aldehyde groups" in Ann Thorac Surg 1988;46:309-16.

Ferner wurden Versuche vorgenommen, eine Detoxifizierung mit Hilfe von Zitronensäure zu erreichen. Dies ist offenbart in Gulbins H., Goldemund A., Anderson I., Haas U., Uhlig A., Meiser B., Reichart B. "Preseeding with autologous fibroblasts improves endothelialization of glutaraldehydefixed porcine aortic valves" in J Thorac Cardiovasc Surg 2003; 125:592-601 teilweise gelungen..

Die dort erzielten Detoxifizierungsgrade betrugen lediglich 20 bis 30 %.

Bei einer geeigneten Detoxifizierung der mit Glutaraldehyd fixierten und einer entsprechenden Beschichtbarkeit durch körpereigenes Gewebe nach der Implantation - der sogenannten "Endothelialisierung" - könnte das Ziel einer lebenslang haltbaren Herzklappe ohne Medikamentengabe zur Blutgerinnungshemmung erreicht werden.

Ein weiteres Beispiel für die Bandbreite der vorliegenden Erfindung sind kleinlumige Gefäßprothesen. Derartige Implantate werden derzeit üblicherweise aus Kunststoff, nämlich PTFE oder PET hergestellt. Sie Besitzen eine vergleichsweise hohe Verschlussrate insbesondere bei ihrer Anwendung als Gefäßersatz peripherer Beingefäße, aortokoronarer Bypässe und peripherer Dialyseshunts. Die Folgen von Gefäßverschlüssen in diesen Prothesenbereichen sind mit Beinamputation, Myokardinfarkt mit Todesfolge oder die Notwendigkeit einer Shuntrevision drastisch. Auch hier könnten kleinlumige, glutaraldehydfixierte biologische Gewebe tierischen Ursprungs in Form von Spendergefäßen des Schweins, Rinds oder der Ziege eine erhebliche Verbesserung bringen, wenn derartige Gewebe detoxifiziert und endothelialisiert werden könnten oder sich auch im Blutstrom selbst endothelialisieren. Auch in der Anwendung von muskulären Pumpen könnten sich thromboembolische Komplikationen vermeiden lassen, wenn die mit dem Blut in Kontakt tretenden Komponenten in ihrer Biokompatibilität, beispielsweise durch geeignete Detoxifizierungsmaßnahmen gesteigert werden könnten.

Weitere Anwendungsbereiche solcher biologischer Gewebe in Form kollagener Matrices tierischen Ursprungs können stabile und biokompatible, glutaraldehydfixierte und detoxifizierte biologische Gewebe zur Arthrosebehandlung von Hüft,- Knie- und Sprunggelenken sein. Ferner sind weitere Anwendungen zum Verschluss des Hirnschädels nach Verletzungen oder Tumoroperationen mit Hilfe eines entsprechend glutaraldehydfixierten und detoxifizierten Rinderperikards als zerebroprotektive Anwendung genau so denkbar, wie Anwendungen beispielsweise in der Thoraxchirurgie als Thoraxwand oder Zwerchfellersatz, in der Abdominalchirurgie zum Bauchwandersatz oder im HNO-Bereich als Trommelfellersatz.

Die WO 01/91820 A1 offenbart ein Verfahren zur Herstellung eines bioartifiziellen Transplantats, das für den Herzklappenersatz beim Menschen verwendet werden soll. Dabei werden solche Transplantate tierischer Herkunft chemisch behandelt und zwar mit Glutaraldehyd.

Als Problematik der Glutaraldehyd-Behandlung eines solchen tierischen Gewebes wird in diesem Dokument die stetige Verhärtung und fortschreitende Calzifizierung nach der Transplantation genannt. Nicht erwähnt ist die Toxizität der Glutaraldehyd-behandelten tierischen Gewebe. Vielmehr liegt der dort offenbarten Erfindung demgegenüber das Problem zugrunde, für den Transplantations-Empfänger fremdes biologisches Gewebe zu einem empfängerverträglichen, immunologisch akzeptablen, bioartifiziellen Transplantat umzuformen, was mithilfe eines Verfahrens realisiert werden soll, das mechanisch stabilere, naturähnlichere Transplantate erzeugt.

Die in diesem Stand der Technik vorgesehene Lösung dieser Aufgabe zielt darauf ab, dem in nativer Form vorliegenden Gewebe in einem konditionierenden Medium empfängerverträgliche Zellen zuzuführen, die ausgewählte, zu einem Re-modelling der Trägerstruktur befähigte Zellen umfassen, wobei die Behandlung des Transplantats fortgesetzt wird, bis eine weitgehende Umbildung des ursprünglichen nativen Gewebes in einem wesentlichen die zugeführten empfängerspezifischen Zellen aufweisendes Gewebe erzielt worden ist.

Vom Grundansatz her soll also keine chemische oder "mechanische" (im Sinne einer Beschichtung) Behandlung des biologischen Gewebes stattfinden, sondern ein komplexer Umbau der Gewebestruktur.

Die im oben genannten Dokument WO 01/91820 A1 offenbarte Plasmabehandlung ist nun lediglich zum Zwecke der Sterilisierung der verwendeten und behandelten Gewebe erwähnt, was nichts mit einer Detoxifizierung der Glutaraldehyd-stabilisierten Gewebe zur Steigerung der Bioverträglich zu tun hat. Bei dieser Sterilisation wird eine Plasmaionisation in Anwesenheit von Wasserstoffperoxid (H₂O₂) durchgeführt.

Die WO 2006/077582 A2 offenbart ein System und ein Verfahren zur Behandlung biologischen Gewebes mit einer Plasmagasentladung, wobei sich diese Plasmabehandlung auf kosmetische Effekte bezieht. Insoweit betrifft dieses Dokument in keiner Weise die Aufbereitung von Implantatgeweben, geschweige denn die Problematik der Toxizität eines Glutaraldehyd-stabilisierten Implantats.

Die US 6 093 530 offenbart ein Verfahren zur Behandlung biologischer Gewebe nach Glutaraldehyd-Fixierung. Um die Calcifizierung nach der Transplantation zu vermeiden, wird das Gewebe durch Oxidation stabilisiert. Diesem Dokument sind keine Hinweise auf eine Plasmabehandlung, das Aufbringen einer Metallschicht und einer Trocknung des biologischen Gewebes entnehmbar.

Im Hinblick auf die geschilderten Probleme des Standes der Technik liegt der Erfindung die Aufgabe zugrunde, ein Verfahren zur Behandlung kollagener, Glutaraldehyd-behandelter Gewebe tierischen oder menschlichen Ursprungs sowie ein entsprechend behandeltes biologisches Gewebe anzugeben, wobei die Bioverträglichkeit und Langzeitstabilität des Gewebes so gesteigert wird, dass die Einsatzzeit des Gewebes im Körper drastisch - im Optimalfall bis zur Dauereinsatzfähigkeit - erhöht werden kann.

Diese Aufgabe wird in verfahrenstechnischer Hinsicht durch die im Kennzeichnungsteil des Anspruches 1 angegebenen Verfahrensschritte gelöst. Ein entsprechendes biologisches Gewebe ist erfindungsgemäß nach Anspruch 7 vorgesehen.

Die grundsätzliche Konzeption der Erfindung setzt zur Lösung dieser Problematik auf eine an sich bekannte physikalische Plasmagasbehandlung des biologischen Gewebes auf, bei dem vorzugsweise Sauerstoff als angeregtes Gas verwendet wird. Aber auch Stickstoff, Wasserstoff und Argon können eingesetzt werden.

Versuche haben gezeigt, dass durch diese physikalische Plasmabehandlung, bei der die eingesetzten Gase angeregt und radikalisiert werden, die die Toxizität aufgrund der Glutaraldehydfixierung erzeugten freien Aldehydgruppen chemisch neutralisiert und damit als erster Schritt für einen zur Besserung der Implantationseigenschaften des Gewebes eine gegenüber dem Stand der Technik deutliche verbesserte Detoxifizierung erreicht werden können. Die erwähnten Versuche haben einen Detoxifizierungsgrad von über 80 % ergeben. Auf den Blutkontaktflächen der Versuchsgewebe konnte dadurch ein geschlossener Endothelrasen angesiedelt werden.

Der bei der Plasmabehandlung grundsätzlich ablaufende chemische Prozess kann am Beispiel des Prozessgases Sauerstoff kurz umrissen werden. So bilden die im Plasma vorhandenen Komponenten, nämlich Sauerstoffionen und der angeregte Sauerstoff bei der Reaktion mit Kohlenwasserstoffen an der Gewebeoberfläche Kohlendioxid und Wasser. Diese Reaktion kann also zur Entfernung der an den Gewebeoberflächen vorhandenen Aldehydgruppen, wie sie durch die Glutaraldehydfixierung entstehen, im Sinne einer Detoxifizierung herangezogen werden.

Für die Behandlung des biologischen Gewebes mit dem Plasmagasverfahren in einer Behandlungskammer ist es sehr günstig für das Detoxifizierungsergebnis, wenn die in aller Regel wässrigen biologischen Gewebe erfindungsgemäß vor der Plasmabehandlung einer Trocknung insbesondere durch Vakuum- und Temperatureinfluss unterzogen werden.

Damit wird das biologische Gewebe für die Dauer der Behandlung komplett wasserfrei gehalten.

Das der Plasmabehandlung unterzogene Gewebe wird dann mit einer biokompatiblen, metallhaltigen Beschichtung versehen. Diese Beschichtung stellt eine zusätzliche biokompatible Komponente auf den Implantatoberflächen dar, die das Zellwachstum nachhaltig fördert

Anschließend wird dem der Plasmabehandlung unterzogenen Gewebe für den Implantationseinsatz Feuchtigkeit zugeführt, indem es beispielsweise in Flüssigkeit eingelegt wird. Es erhält damit seine ursprüngliche Konsistenz wieder und ist beispielsweise bei einer entsprechenden Realisierung als Schweineherzklappe dauerelastisch und langzeitstabil.

In den Unteransprüchen 2 und 3 sind bevorzugte Verfahrensbedingungen für das eingesetzte Plasmagasverfahren angegeben. Die Energieeinkopplung für die Erzeugung des Plasmas wird vorteilhafterweise durch ein hochfrequentes elektromagnetisches Feld, insbesondere ein Mikrowellenfeld, realisiert.

Die vorstehenden Plasmagasverfahren finden in der Regel durch Einleiten des zu ionisierenden Gases in eine evakuierte Behandlungskammer statt. Daneben ist es auch möglich, die Plasmabehandlung mit einem atmosphärischen Plasma unter Einsatz eines Plasmastrahls mit angeregtem Reaktionsgas unter atmosphärischen Bedingungen durchzuführen. Der Plasmastrahl wird über die Implantatoberfläche geführt, so dass eine lokale Reinigung stattfindet. Diese erfolgt dadurch, dass die im Reaktionsgas vorhandenen Sauerstoffionen mit Kohlenwasserstoffen and der Gewebeoberfläche eine Reaktion eingehen und Kohlendioxid und Wasser bilden.

Eine besonders bevorzugte Weiterbildung des erfindungsgemäßen Verfahrens ist in den Ansprüchen 5 und 6 angegeben. Das Verfahren der Wahl zur Realisierung der metallhaltigen Beschichtung ist ein PACVD-Verfahren, das bei der Beschichtung von Kunststoffoberflächen bei künstlichen medizinischen Implantaten bereits überzeugende Ergebnisse bezüglich der Bioverträglichkeit der damit behandelten Oberflächen erzielt hat. Diese metallhaltige Beschichtung ist ausgewählt aus der Gruppe von Metallen bestehend aus Ti, Ta, Nb, Zr, Hf, Ir, Au, Pd, Pt, Ag und Cu. Besonders bewährt hat sich in diesem Zusammenhang Titan, das als besonders biokompatibles Material in vielerlei Implantatanwendungen seit langer Zeit im Einsatz ist. Die ebenfalls erwähnten Beschichtungsmaterialien Silber und Kupfer können zusätzlich oder ausschließlich als antibakterielle Reagenzien in die Beschichtung eingebracht werden.

Die Ansprüche 7 bis 9 beziehen sich auf biologische Gewebe tierischen oder menschlichen Ursprungs, wie sie als Implantat in einem menschlichen oder auch tierischen Körper eingesetzt werden können. Entsprechend der vorliegenden Erfindung ist mindestens die mit dem Körper in Kontakt bringbare Oberfläche nach Trocknung des Glutaraldehyd-behandelten Gewebes einer Plasmabehandlung zur Detoxifizierung unterzogen, wobei die plasmabehandelte Oberfläche mit einer biokompatiblen, metallhaltigen Beschichtung versehen und das Gewebe einer Feuchtigkeitszuführung unterzogen ist. Die Biokompatibilität wird - wie oben erörtert - durch eine biokompatible, metallhaltige Beschichtung auf dem biologischen Gewebe verbessert. Bevorzugtermaßen handelt es sich bei dem biologischen Gewebe um eine Herzklappe, Gefäßprothese, Blutkontaktfläche mechanischer oder biomechanischer Blutpumpen, Verschlusseinsatz für Hirnschädelöff nungen, Knorpel-, Knochen-, Sehnen-, Zwerchfell-, Thoraxwand-, Bauchwand- oder Trommelfellersatz.

Die folgende Beschreibung erläutert die Erfindung näher in einem

### Ausführungsbeispiel:

Als Beispiel für ein kollagenes Gewebe tierischen Ursprungs, das als Implantat in einen menschlichen Körper eingesetzt werden soll, wird eine Schweineherzklappe herangezogen.

Diese wird nach der Entnahme aus dem Spendertier zur Stabilisierung in üblicher Weise präpariert, dezellularisiert und glutaraldehydfixiert. Dabei wird die Klappe in Glutaraldehydlösung zwischen 0,1 und 0,4% gebracht und in strömender Lösung bei geringen Drucken von 3-6 mm Hg über 24-48 Stunden fixiert.

Die so präparierte Schweineherzklappe wird dann unter Vakuum und Zuführung von Temperatur langsam getrocknet und damit komplett entwässert.

Danach wird in einer Behandlungskammer eine Plasmagasbehandlung der Schweineherzklappe vorgenommen. Dazu wird die Behandlungskammer vollständig evakuiert und anschließend Sauerstoff eingeleitet. Ein Plasma wird durch Einkoppeln eines hochfrequenten elektromagnetischen Feldes von beispielsweise von 40 kHz oder 13,56 MHz oder durch Anregung mit Mikrowellen gezündet. Durch die damit verbundene Energiezufuhr wird das in der Behandlungskammer vorhandene Sauerstoffgas angeregt und radikalisiert.

Dieses Plasmagas wirkt auf die Kohlenwasserstoffgruppen CₓH_{y} an der Oberfläche des glutaraldehydfixierten Implantats gemäß folgender Reaktionsgleichung:

CₓH_{y}+(x+y/4)O₂ → x CO₂+y/2 H₂O.

Erkennbarer Weise werden also Kohlenwasserstoffe an der Implantatoberfläche, wie beispielsweise Acetaldehyde in die vergleichsweise harmlosen chemischen Verbindungen Kohlendioxid und Wasser umgewandelt, die leicht von der Implantatoberfläche entfernt werden können.

Die vorstehende Plasmabehandlung wird dann zum Aufbringen der metallhaltigen Beschichtung auf der Implantatoberfläche weitergeführt. Dazu wird in die Beschichtungskammer ein gasförmiger Precursor zugeführt, der sich unter dem Einfluss der Plasmaenergie in seine atomaren Bestandteile zerlegt. Die so entstehenden Ionen lagern sich an der Oberfläche an. Typischerweise wird als metallhaltige Beschichtung in erster Linie Titan mit Hilfe des PACVD-Verfahrens aufgebracht. Das Verfahren als solches ist im Übrigen in der EP 0 897 997 B1 am Beispiel der Beschichtung eines Kunststoffsubstrats ausführlich beschrieben.

Der Reaktordruck sowohl für die Vorbehandlung als auch für die Beschichtung liegt zwischen 0,1 und 1030 mbar. Bei Anwendung eines Plasmas sollte der Druck idealerweise >50 mbar betragen. Für die Vorbehandlung wird das Arbeitsgas (z.B. Sauerstoff) mit einem Gasvolumenstrom von 0,04 Nl/min. in den Reaktor eingeleitet. Nach Stabilisierung des Enddruckes auf ca. 1 mbar erfolgt die kapazitive Plasmaeinkopplung mit einer Leistung von 20 W für eine Dauer von 60 sec. Anschließend wird die Gaszufuhr unterbrochen und die Reaktorkammer komplett evakuiert. Für die nachfolgende Beschichtung wird das Trägergas (Wasserstoff) mit einem Gasvolumenstrom von 0,09 Nl/min über den Precursor Ti[N(CH₃)₂]₄ geleitet und in die Beschichtungskammer eingebracht. Die Beschichtungsdauer beträgt ca. 300 sec. mit einer eingestellten Plasmaleistung von 20 W. Anschließend wird die Gaszufuhr wieder abgestellt und die Beschichtungskammer belüftet.

Nach dieser Vakuumbehandlung zur Plasmabeaufschlagung und Beschichtung der Schweine-Herzklappen werden diese wieder in Flüssigkeit eingelegt, so dass sie durch die zugeführte Feuchtigkeit ihre ursprüngliche Konsistenz wieder erhalten.

## Patentansprüche

1. Verfahren zur Behandlung biologischer Gewebe tierischen oder menschlichen Ursprungs, wie Schweine-, Rinderperikard- oder menschliche Leichen-Herzklappen, zur Steigerung der Bioverträglichkeit, basierend auf einer physikalischen Plasmabehandlung des Glutaraldehyd-behandelten Gewebes durch ein Plasmagasverfahren, bei dem als ionisiertes Gas Stickstoff, Wasserstoff, Argon oder Sauerstoff verwendet wird,
**dadurch gekennzeichnet, dass**
- vor der Behandlung mittels des Plasmagasverfahrens das Glutaraldehyd-behandelte Gewebe getrocknet wird,
- das der Plasmabehandlung unterzogene, getrocknete Gewebe mit einer biokompatiblen, metallhaltigen Beschichtung versehen wird, und
- dem getrockneten Implantat nach der Plasmabehandlung Feuchtigkeit zugeführt wird.

2. Verfahren nach Anspruch 1, **dadurch gekennzeichnet, dass** die Energieeinkopplung für die Erzeugung des Plasmas durch ein hochfrequentes elektromagnetisches Feld, insbesondere ein Mikrowellenfeld erzeugt wird.

3. Verfahren nach Anspruch 1, **dadurch gekennzeichnet, dass** die Plasmabehandlung mit einem atmosphärischen Plasma unter Einsatz eines Plasmastrahles unter atmosphärischen Bedingungen erfolgt.

4. Verfahren nach Anspruch 1, **dadurch gekennzeichnet, dass** die Trocknung durch Vakuum- und Temperatureinfluss erfolgt.

5. Verfahren nach Anspruch 1, **dadurch gekennzeichnet, dass** die metallhaltige Beschichtung mittels eines PACVD-Verfahrens aufgebracht wird.

6. Verfahren nach einem der vorgenannten Ansprüche, **dadurch gekennzeichnet, dass** die metallhaltige Beschichtung ausgewählt ist aus der Gruppe bestehend aus Ti, Ta, Nb, Zr, Hf, Ir, Au, Pd, Pt, Ag und Cu.

7. Biologisches Gewebe tierischen oder menschlichen Ursprungs zum Einsatz als Implantat in einem menschlichen oder tierischen Körper, hergestellt mit einem Verfahren nach einem der vorgenannten Ansprüche, **dadurch gekennzeichnet, dass** mindestens seine mit dem Körper in Kontakt bringbare Oberfläche nach Trocknung des Glutaraldehyd-behandelten Gewebes einer Plasmabehandlung zur Detoxifizierung unterzogen, die plasmabehandelte Oberfläche mit einer biokompatiblen, metallhaltigen Beschichtung versehen und das Gewebe einer Feuchtigkeitszuführung unterzogen ist.

8. Biologisches Gewebe nach Anspruch 7, **dadurch gekennzeichnet, dass** die metallhaltige Beschichtung ausgewählt ist aus der Gruppe bestehend aus Ti, Ta, Nb, Zr, Hf, Ir, Au, Pd, Pt, Ag und Cu.

9. Biologisches Gewebe nach Anspruch 7 oder 8, **dadurch gekennzeichnet, dass** es als Herzklappe, Gefäßprothese, Blutkontaktfläche mechanischer oder biomechanischer Blutpumpen, Verschlusseinsatz für Hirnschädelöffnungen, Knorpel-, Knochen-, Sehnen-, Zwerchfell-, Thoraxwand-, Bauchwand- oder Trommelfellersatz ausgebildet ist.

## Claims

1. Method for the treatment of biological tissue of animal or human origin, such as porcine, bovine pericardium or human cadaver heart valves, to increase the biocompatibility based on a physical plasma treatment of the tissue treated with glutaraldehyde by a plasma gas method in which nitrogen, hydrogen, argon or preferably oxygen is used as the ionised gas,
**characterised in that**
- the tissue treated with glutaraldehyde is dried before the treatment by means of the plasma gas method,
- the tissue subjected to the plasma treatment is provided with a biocompatible, metal-containing coating, and
- moisture is supplied to the dried implant after the plasma treatment.

2. Method according to claim 1, **characterised in that** the energy input for the production of the plasma takes place by means of a high-frequency electromagnetic field, in particular a microwave field.

3. Method according to claim 1, **characterised in that** the plasma treatment takes place with an atmospheric plasma using a plasma jet under atmospheric conditions.

4. Method according to claim 1, **characterised in that** the drying takes place by means of the influence of a vacuum and temperature.

5. Method according to claim 1, **characterised in that** the metal-containing coating is applied by means of a PACVD method.

6. Method according to one of the preceding claims, **characterised in that** the metal-containing coating is selected from the group consisting of Ti, Ta, Nb, Zr, Hf, Ir, Au, Pd, Pt, Ag and Cu.

7. Biological tissue of animal or human origin for use as an implant in a human or animal body, produced with a method according to one of the preceding claims, **characterised in that** at least its surface that can be brought into contact with the body, after drying the tissue treated with glutaraldehyde, is subjected to a plasma treatment for detoxification, the plasma-treated surface is provided with a biocompatible, metal-containing coating and the tissue is subjected to a moisture supply.

8. Biological tissue according to claim 7, **characterised in that** the metal-containing coating is selected from the group consisting of Ti, Ta, Nb, Zr, Hf, Ir, Au, Pd, Pt, Ag and Cu.

9. Biological tissue according to claim 7 or 8, **characterised in that it** is configured as a heart valve, vessel prosthesis, blood contact face of mechanical or biomechanical blood pumps, closure insert for cranium openings, or the replacement of cartilage, bone, tendons, the diaphragm, thorax wall, abdominal wall or eardrum.

## Revendications

1. Procédé de traitement de tissus biologiques d'origine animale ou humaine, tels que des valves péricardiques de porc, de bovin, ou des valves cardiaques de cadavres humains, pour l'amélioration de la biocompatibilité, basé sur un traitement physique à l'aide d'un procédé par plasma de tissus traités au glutaraldéhyde par un procédé par plasma gazeux, dans lequel on utilise de l'azote, de l'hydrogène, de l'argon ou de l'oxygène en tant que gaz ionisé,
**caractérisé en ce**
- **qu**'avant le traitement au moyen du procédé par plasma gazeux, le tissu traité au glutaraldéhyde est séché,
- **que** le tissu, soumis au traitement par plasma, séché, est doté d'un revêtement biocompatible, contenant du métal et
- **que** de l'humidité est apportée sur l'implant séché après le traitement au plasma.

2. Procédé selon la revendication 1, **caractérisé en ce que** le couplage d'énergie pour la genèse du plasma est généré par un champ électromagnétique de haute fréquence, notamment un champ de micro-ondes.

3. Procédé selon la revendication 1, **caractérisé en ce que** le traitement par plasma est effectué avec un plasma atmosphérique moyennant l'emploi d'un rayonnement plasma dans des conditions atmosphériques.

4. Procédé selon la revendication 1, **caractérisé en ce que** le séchage est réalisé par l'influence du vide et de la température.

5. Procédé selon la revendication 1, **caractérisé en ce que** le revêtement contenant du métal est rapporté au moyen d'un procédé de dépôt chimique en phase vapeur assisté par plasma PACVD.

6. Procédé selon l'une des revendications précitées, **caractérisé en ce que** le revêtement contenant du métal est choisi dans le groupe constitué par Ti, Ta, Nb, Zr, Hf, Ir, Au, Pd, Pt, Ag et Cu.

7. Tissu biologique d'origine animale ou humaine pour une utilisation en tant qu'implant dans un corps humain ou animal, fabriqué à l'aide d'un procédé selon l'une des revendications précitées, **caractérisé en ce qu**'au moins sa surface pouvant être mise en contact avec le corps après un séchage du tissu traité au glutaraldéhyde dans un traitement par plasma est soumis à une détoxification, la surface traitée par plasma est pourvue d'un revêtement biocompatible contenant du métal et le tissu est soumis à un passage dans l'humidité.

8. Tissu biologique selon la revendication 7, **caractérisé en ce que** le revêtement contenant du métal est choisi dans le groupe constitué par Ti, Ta, Nb, Zr, Hf, Ir, Au, Pd, Pt, Ag et Cu.

9. Tissu biologique selon les revendications 7 ou 8, **caractérisé en ce qu**'il est conçu sous forme de valve cardiaque, de prothèse vasculaire, de surface de contact avec le sang pour des pompes de sang mécaniques ou biomécaniques, de produits de comblement pour des ouvertures dans la boîte crânienne, de substituts de cartilage, d'os, de ligaments, de diaphragme, de paroi thoracique, de paroi abdominale ou de tympan.
